(19) Europäisches Patentamt
European Patent Office
Office européen des brevets

(11) Publication number: 0 001 688
B1

(12) **EUROPEAN PATENT SPECIFICATION**

(45) Date of publication of patent specification: 04.11.81

(21) Application number: 78300435.1

(22) Date of filing: 29.09.78

(51) Int. Cl.$^3$: C 07 D 493/22, A 01 N 43/22, C 07 H 17/08

# (54) Derivatives of C-076 compounds and their preparation.

(30) Priority: 03.10.77 US 839099
03.10.77 US 838602

(43) Date of publication of application:
02.05.79 Bulletin 79/9

(45) Publication of the grant of the European patent:
04.11.81 Bulletin 81/44

(84) Designated Contracting States:
BE CH DE FR GB LU NL SE

(56) References cited:

DE - A - 2 329 486
DE - A - 2 717 040

Chemical Abstracts vol. 83, no 15, 13. October 1975, Columbus, Ohio, USA,
A. AOKI et al "Antibiotic B-41 A, derivatives from Streptomyces as acaricides" page 163, abstract no. 127 530 s

(73) Proprietor: MERCK & CO. INC.
126, East Lincoln Avenue P.O. Box 2000
Rahway, New Jersey 07065 (US)

(72) Inventor: Mrozik, Helmut Hugo
159 Idlebrook Lane
Matawan New Jersey 07747 (US)
Inventor: Fisher, Michael Herbert
1140 Concord Drive
Bridgewater New Jersey 08807 (US)
Inventor: Kulsa, Peter
933 Belvidere Avenue
Plainfield New Jersey 07060 (US)
Inventor: Albers-Schonberg, George
30 Tyson Lane
Princeton New Jersey 08540 (US)

(74) Representative: Crampton, Keith John Allen et al,
D YOUNG & CO 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 001 688 B1

# Derivatives of C-076 compounds and their preparation

This invention relates to C-076.

The term C-076 is used to describe a series of compounds isolated from the fermentation broth of a C-076-producing strain of *Streptomyces avermitilis,* as disclosed in our German published Patent Specification No. 2,717,040. The morphological characteristics of the culture are completely described below. The C-076 compounds are a series of macrolides with hydroxy substituents capable of being acylated. Some of the C-076 compounds have more than one acylable hydroxy group, and procedures have been developed for the selective acylation at the various positions. The acyl compounds thus produced have profound anthelmintic, insecticidal, ectoparasiticidal and acaricidal activity.

## SUMMARY OF THE INVENTION

The C-076 series of compounds have the following structure:

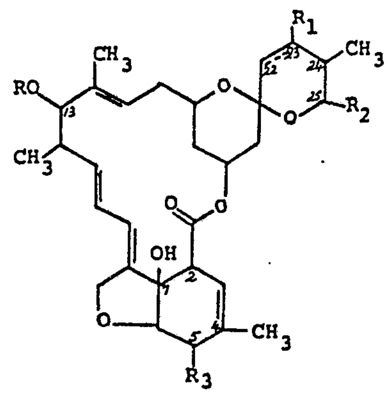

wherein R is the α-L-oleandrosyl-α-L-oleandrosyl group of the structure:

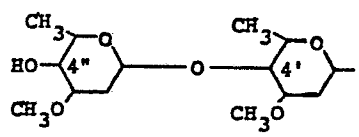

and wherein the broken line between $C_{22}$ and $C_{23}$ indicates that the linkage is saturated or ethylenically unsaturated; $R_1$ is hydroxy and is present only when said broken line indicates that the said linkage is saturated;

$R_2$ is *iso*-propyl or *sec*-butyl; and

$R_3$ is methoxy or hydroxy.

There are eight different C-076 compounds and they are given the designations A1a, A1b, A2a, A2b, B1a, B1b, B2a, B2b based upon the structure of the individual compounds.

In the foregoing structural formula, the individual C-076 compounds are as set forth below.

| | $R_1$ | $R_2$ | $R_3$ |
|---|---|---|---|
| A1a | Double bond | *sec*-butyl | $—OCH_3$ |
| A1b | Double bond | *iso*-propyl | $—OCH_3$ |
| A2a | —OH | *sec*-butyl | $—OCH_3$ |
| A2b | —OH | *iso*-propyl | $—OCH_3$ |
| B1a | Double bond | *sec*-butyl | —OH |
| B1b | Double bond | *iso*-propyl | —OH |
| B2a | —OH | *sec*-butyl | —OH |
| B2b | —OH | *iso*-propyl | —OH |

As is readily seen, all of the C-076 compounds have hydroxy groups at the 7-position and the 4″-position of the carbohydrate side chain. The 7-position hydroxy group, however, is resistant to acylation and under the conditions described herein, no 7-position acylation product having been isolated. Thus all of the compounds have at least one acylatable hydroxy group. In addition, the A2 and B1 series of compounds have a second acylatable hydroxy group and the B2 series of compounds has a third acylatable hydroxy group.

The carbohydrate side chain may also be hydrolyzed to remove one or both of the α-L-oleandrose groups. In this case there would remain an acylatable hydroxy group at the 4′ or 13-position with the removal of a single α-L-oleandrose (monosaccharide) or both α-L-oleandrose (aglycone) respectively. The monosaccharide and aglycone compounds are active compounds and part of this invention.

The monosaccharide and aglycone derivatives are prepared by the treatment of the parent C-076 compound with acid. The outer α-L-oleandrose group is more easily removed than the α-L-oleandrose group directly bonded to the C-076 substrate thus facilitating the separate preparation of the monosaccharide and aglycone without contamination with the other reaction product.

The process employed for the removal of the C-076 carbohydrate group or groups is to put the C-076 starting material in solution in a mixture of from 0.01 to 10% acid in a non-nucleophilic water miscible solvent such as dioxane, tetrahydrofuran, dimethoxyethane, dimethylformamide or *bis*-2-methoxyethyl ether, and from 0.1 to 20% water. The mixture is stirred for from 6 to 24 hours at room temperature to complete the reaction. Acids such as sulfuric, hydrochloric, hydrobromic, phosphoric, trifluoroacetic and trifluorosulfonic are acceptable. Sulfuric acid is preferred.

When lower acid concentrations are used such as from 0.01 to 0.1% are employed the monosaccharide is predominantly prepared. When higher concentrations of acid are employed, such as in the range of 1 to 10%, the aglycone is predominantly prepared. Intermediate concentrations of acid will tend to prepare mixtures of monosaccharide and aglycone which are generally separable using chromatographic techniques.

The monosaccharide may also be prepared by stirring the C-076 precursor for from 6 to 24 hours at room temperature in 1% sulfuric acid in isopropanol. In addition the aglycone can be prepared by stirring the C-076 precursor for from 6 to 24 hours at room temperature in 1% sulfuric acid in methanol. The other acids listed above may also be employed in this process. This process is preferred for use with the 2- series of C-076 compounds since some addition may be observed to the 22, 23 double bond of the series of C-076 compounds with a 22, 23 unsaturation. The desired monosaccharide or aglycone are isolated and purified using techniques known to those skilled in the art.

The compounds of this invention are realized in the following structural formula:

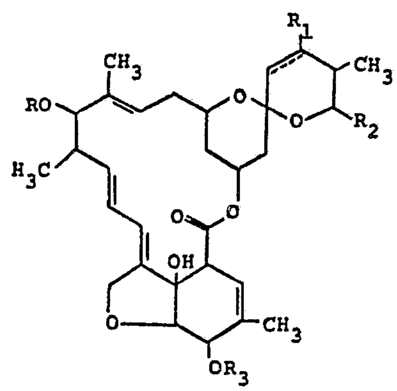

wherein the broken line indicates that the linkage is saturated or ethylenically unsaturated;

$R_1$ is hydroxy or acyloxy and is present only when the broken line indicates that the said linkage is saturated;

$R_2$ is *iso*-propyl or *sec*-butyl;

$R_3$ hydrogen, methyl or acyl; and

R is hydrogen, acyl, α-L-oleandrosyl, 4′-acyl-α-L-oleandrosyl, 4′-(α-L-oleandrosyl)-α-L-oleandrosyl, 4″-acyl-4′-(α-L-oleandrosyl)-α-L-oleandrosyl;

provided that when R is α-L-oleandrosyl-α-L-oleandrosyl at least one of the $R_1$, or $R_3$ groups contains an acyl group.

The foregoing acyl groups and the acyl portion of the acyloxy groups are: loweralkanoyl; substituted loweralkanoyl wherein the substituents are halogen, carboxy, loweralkoxycarbonyl, amino, mono- or di-loweralkylamino, or loweralkanoylamino; unsaturated loweralkanoyl, loweralkoxycarbonyl, halogenated loweralkoxycarbonyl, benzoyl, or substituted benzoyl in which the substituents are halogen, nitro, alkyl, amino, hydroxy or alkoxy; carbamoyl and N-substituted and N,N-disubstituted carbamoyl wherein the substitution is loweralkyl, benzyl, hydroxyloweralkyl, or the carbamoyl nitrogen may be incorporated into a morpholine heterocycle. The acyloxy group at $R_1$ is defined as the above acyl groups bonded to the 23-position through an oxygen atom.

In the present application the term "loweralkyl" means those straight or branched chain alkyl

groups containing from 1 to 12 carbon atoms such as methyl, ethyl, propyl, isopropyl, butyl, pentyl, hexyl, decyl and dodecyl.

The term "loweralkoxy" means those alkoxy groups containing from 1 to 6 carbon atoms such as methoxy, ethoxy, propoxy, isopropoxy, butoxy, tert-butoxy, pentoxy and hexoxy.

The term "loweralkanoyl" means those saturated alkanoyl groups containing from 2 to 12 carbon atoms such as acetyl, propionyl, butyryl, pentanoyl, hexanoyl, pivaloyl, octanoyl and decanoyl.

"Unsaturated loweralkanoyl" means those alkanoyl groups containing from 3 to 12 carbon atoms and a double bond such as acryloyl and crotonoyl.

The term "halogen" means the halogen atoms fluorine, chlorine, bromine, and iodine.

The carbamoyl group is defined as the following:

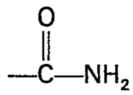

with optional substitution or disubstitution on the nitrogen atom.

The most preferred substitution is a loweralkanoyl group at the 4″ position, the 5-position, the 4″ and 5 positions or the 4″ and 23 positions. The acetyl group is the most preferred loweralkanoyl group.

The acylated compounds are prepared using acylation techniques in which the reaction conditions will vary, depending upon the reactivity of the hydroxy group being acylated. Where there is more than one hydroxy group to be acylated, different reaction conditions are employed to minimize the formation of mixtures.

The acylation reagents employed are generally the halide, preferably the chloride, of the above named acyl groups. Thus the acyl halide reagent in the case of the loweralkanoyl, substituted loweralkanoyl and unsaturated loweralkanoyl acyl groups would be the loweralkanoyl, substituted loweralkanoyl or unsaturated loweralkanoyl halide. Similarly the loweralkoxycarbonyl halide; benzoyl or substituted benzoyl halide; carbamoyl or substituted carbamoyl halide could be employed to form the respective acylated compound. The acyl haloformate, preferably the chloroformate is another successful acylation reagent.

In addition, in the case of the loweralkanoyl, substituted loweralkanoyl, benzoyl or substituted benzoyl groups, the acylation reagent could be in the form of the anhydride. In the case of reactions carried out with the halide reagents it is often advantageous to include in the reaction mixture a basic compound capable of reacting with and neutralizing the hydrogen halide which is liberated during the course of the reaction. Tertiary amines are preferred such as triethylamine, pyridine, 4-dimethylamino pyridine and diisopropyl ethylamine. The basic compound is required in equimolar amounts relative to the numbered moles of hydrogen halide being liberated, however excess amounts, even using the basic compound as a solvent, are not detrimental.

In the case of the A1 compounds, there is only a single hydroxy group, 4″ hydroxy, which may be acylated. The formation of the monosaccharide or the aglycone still leaves only a single hydroxy group which may be acylated, that is the 4′ or 13 hydroxy group.

In the case of the 4″, 4′ and 13 hydroxy groups of C-076 A1 compounds, the acylating reagent is dissolved in a suitable solvent, pyridine is preferred, and the acylating reagent added. The reaction is maintained at from 0°C to room temperature for from 4 to 24 hours. The product is isolated using known techniques.

The A2 compounds have two available hydroxy groups, the 4″ (4′ or 13) or the 23 positions. The different hydroxy groups may be selectively acylated by controlling the reaction conditions.

The 4″ (4′ or 13) monoacyl compound may be prepared by using the reaction conditions described above for the A1 compound. Since the 23 hydroxy is less reactive than the 4″ (4′ or 13) position, mild reaction conditions (0°C) will afford predominantly the monoacyl compound. Heating the reaction mixture at from room temperature to 100°C for from 1 to 24 hours will produce the 4″ (4′ or 13), 23-diacyl compound. If the 23 monoacyl compound is desired, the diacyl compound is treated with aqueous base, such as sodium hydroxide, at room temperature for from 1 to 24 hours. The 4″ (4′ or 13) acyl group will be hydrolyzed leaving the 23 monoacyl compound.

The B1 compounds also have 2 available hydroxy groups: at the 4″ (4′ or 13) and the 5-positions. However, in this case the two hydroxy groups have similiar reactivities. When the reaction of the acylating agent in pyridine is carried out at about room temperature for from 4 to 24 hours, the diacyl compound is recovered. When the reaction is carried out at 0°C a mixture of the 4″ (4′ or 13) and 5 monoacyl compounds are recovered. To recover individual compounds, the mixture is placed on a chromatographic column or a preparative layer chromatographic plate of alumina or silica gel and the individual compounds are readily isolated. In addition, techniques such as high pressure liquid chromatography may be employed to separate mixtures of acylated compounds.

The B2 compounds have three hydroxy groups available for substitution: the 4″ (4′ or 13), 5 and 23 positions. The relative reactivity of the various hydroxy groups is the same as in the other series of compounds. Thus, the triacyl compound may be prepared by carrying out the reaction at from room

temperature to 100°C. The 4″ (4′ or 13), 5 diacyl compound may be prepared by carrying out the reaction at no more than room temperature. At 0°C, a mixture of 4″ (4′ or 13), and 5 monoacyl compounds is recovered which is separable as described above. By varying the reaction conditions and sequence, and by hydrolyzing the undesired acyl groups, all combinations of mono and diacyl compound may be recovered. For example, to prepare the 23-acyl compound, the triacyl compound is hydrolyzed with aqueous base as described above to remove the 4″ (4′ or 13) and 5 acyl groups. Acylation of the 23 monoacyl compound at 0°C will result in a mixture of the diacyl compounds which is readily separable.

The above described acyl compounds are isolated from the reaction mixture using techniques known to those skilled in this art.

The novel acylated compounds of this invention and the monosaccharide and aglycone have significant parasiticidal activity as anthelmintics, insecticides, ectoparasiticides and acaricides, in human and animal health and in agriculture.

The disease or group of diseases described generally as heminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are *Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris.* Certain of these, such as *Nematodirus, Cooperia,* and *Oesophagostomum* attack primarily the intestinal tract while others, such as *Haemonchus* and *Ostertagia,* are more prevalent in the stomach while still others such as *Dictyocaulus* are found in the lungs. Still other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs and, if left untreated, may result in death of the infected host. The C-076 compounds of this invention have unexpectedly high activity against these parasites, and in addition are also active against *Dirofilaria* in dogs, *Nematospiroides, Syphacia, Aspiculuris* in rodents, arthropod ectoparasites of animals and birds such as ticks, mites, lice, fleas, blowfly, in sheep *lucilia sp.,* biting insects and such migrating dipterous larvae as *Hypoderma sp.* in cattle, *Gastrophilus* in horses, and *Cuterebra sp.* in rodents.

The compounds of the present invention are also useful against parasites which infect humans. The most common genera of parasites of the gastro-intestinal tract of man are *Ancylostoma, Necator, Ascaris, Strongyloides, Trichinella, Capillaria, Trichuris,* and *Enterobius.* Other medically important genera of parasites which are found in the blood or other tissues and organs outside the gastrointestinal tract are the filiarial worms such as *Wuchereria, Brugia, Onchocerca* and *Loa, Dracunculus* and extra intestinal stages of the intestinal worms *Strongyloides* and *Trichinella.* The compounds are also of value against arthropods parasitizing man, biting insects and other dipterous pests causing annoyance to man.

The compounds are also active against household pests such as the cockroach, *Blatella sp.,* clothes moth, *Tineola sp.,* carpet beetle, *Attagenus sp.* and the housefly *Musca domestica.*

The compounds are also useful against insect pests of stored grains such as *Tribolium sp., Tenebrio sp.* and of agricultural plants such as spider *mites, (Tetranychus sp.),* aphids, *(Acyrthiosiphon sp.);* against migratory orthopterans such as locusts and immature stages of insects living on plant tissue. The compounds are useful as a nematocide for the control of soil nematodes and plant parasites such as *Meloidogyne spp.* which may be of importance in agriculture.

These compounds may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contains from 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or di-calcium phosphate.

Where it is desired to administer the C-076 compounds in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate and vegetable gums. Such unit dosage formulations may be varied widely with respect to their total weight and content of the antiparasitic agent depending upon factors such as the type of host animal to be treated, the severity and type of infection and the weight of the host.

When the active compound is to be administered in an animal feedstuff, it is intimately dispersed in the feed or used as a top dressing or in the form of pellets which may then be added to the finished

feed or optionally fed separately. Alternatively, the antiparasitic compounds of our invention may be administered to animals parenterally, for example, by intraruminal, intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil or cotton seed oil. Other parenteral vehicles such as organic preparations using solketal, glycerol-formal and aqueous parenteral formulations are also used. The active C-076 compound or compounds are dissolved or suspended in the parenteral formulation for administration; such formulations generally contain from 0.005 to 5% by weight of the active compound.

Although the antiparasitic agents of this invention find their primary use in the treatment and/or prevention of helminthiasis, they are also used in the prevention and treatment of diseases caused by other parasites, for example, orthropod parasites such as ticks, lice, fleas, mites and other biting and sucking insects in domesticated animals and poultry. They are also effective in treatment of parasitic diseases that occur in other animals including humans. The optimum amount to be employed for best results will, of course, depend upon the particular compound employed, the species of animal to be treated and the type and severity of parasitic infection or infestation. Generally, good results are obtained with our novel compounds by the oral administration of from 0.001 to 10 mg. per kg. of animal body weight, such total dose being given at one time or in divided doses over a relatively short period of time such as 1—5 days. With the preferred compounds of the invention, excellent control of such parasites is obtained in animals by administering from 0.025 to 0.5 mg. per kg. of body weight in a single dose. Repeat treatments are given as required to combat re-infections and are dependent upon the species of parasite and the husbandry techniques being employed. The techniques for administering these materials to animals are known to those skilled in the veterinary field. The compounds may also be administered in combination with other antiparasitic compounds or compounds with other biological activities to provide for a single treatment with a broadened spectrum of activity.

When the compounds described herein are administered as a component of the feed of the animals, or dissolved or suspended in the drinking water, compositions are provided in which the active compound or compounds are intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits and crushed limestone. The active C-076 compounds are intimately dispersed throughout the carrier by methods such as grinding, stirring, milling or tumbling. Compositions containing from 0.005 to 2.0% by weight of the active compound are particularly suitable as feed premixes. Feed supplements, which are fed directly to the animal, contain from 0.0002 to 0.3% by weight of the active compounds.

Such supplements are added to the animal feed in an amount to give the finished feed the concentration of active compound desired for the treatment and control of parasitic diseases. Although the desired concentration of active compound will vary depending upon the factors previously mentioned as well as upon the particular C-076 compound employed, the compounds of this invention are usually fed at concentrations of from 0.00001 to 0.002% in the feed in order to achieve the desired antiparasitic result.

In using the compounds of this invention, the individual C-076 compounds may be prepared and used in that form. Alternatively, mixtures of two or more of the individual C-076 compounds may be used.

In the isolation of the C-076 compounds, which serve as starting materials for the processes of the present invention, from the fermentation broth, the various C-076 compounds will be found to have been prepared in unequal amounts. In particular an "a" series compound will be prepared in a higher proportion than the corresponding "b" series compound. The weight ratio of "a" series to the corresponding "b" series is about 85:15 to 99:1. The differences between the "a" series and "b" series is constant throughout the C-076 compounds and consists of a butyl group and a propyl group respectively at the 25 position. This difference, of course does not interfere with any of the present reactions. In particular, it may not be necessary to separate the "b" components from the related "a" component. Separation of these closely related compounds is generally not practised since the "b" compound is present only in a very small percent by weight, and the structural difference has negligible effects on the reaction processes and biological activities.

The C-076 compounds of this invention are also useful in combating agricultural pests that inflict damage upon crops while they are growing or while in storage. The compounds are applied using known techniques such as in sprays, dusts or emulsions, to the growing or stored crops to effect protection from such agricultural pests.

The following examples are provided in order that the invention might be more fully understood; they are not to be construed as limitations of the invention.

The C-076 derivatives prepared in the following examples are generally isolated as amorphous solids and not as crystalline solids. They are thus characterized analytically using technique such as mass spectrometry and nuclear magnetic resonances. Being amorphous, the compounds are not characterized by sharp melting points, however the chromatographic and analytical methods employed indicate that the compounds are pure.

## Example 1

*C-076 A1a 4''-O-Acetate*

A. 0.8 Mg. of C-076 A1a is combined with 3 drops of dry pyridine and 2 drops of acetic anhydride. The reaction mixture is allowed to stand stoppered for 2 days. The solution is transferred to a small flask and washed with benzene. The benzene layer is concentrated *in vacuo*, diluted and concentrated twice more with benzene. A thin layer chromatographic analysis on silica gel using 5% methanol in chloroform reveals only a single spot which is different from the starting material. Preparative layer chromatography on silica gel plates using 10% tetrahydrofuran in chloroform also reveals a single spot with an Rf of 0.5.

B. A solution of 27. mg. of 4-dimethylaminopyridine in 1 ml. of methylene chloride is prepared and a separate solution of 0.208 ml. of acetic anhydride in 10 ml. of methylene chloride is prepared. 0.5 Ml. of each solution is added to 10 mg. of C-076 A1a, mixed well and allowed to stand at room temperature overnight. The reaction mixture is diluted to 4 ml. with methylene chloride and 0.5 ml. of water is added and shaken. The layers are separated and the organic layer is dried over magnesium sulfate and evaporated to dryness under a stream of nitrogen. Benzene is added and the solution is lyophilized affording 10 mg. of an off-white fluffy solid. Preparative layer chromatography on silica gel eluting with 10% tetrahydrofuran in chloroform affords 8.2 mg. of a fluffy, off-white solid, which nuclear magnetic resonance and mass spectrographic analysis reveals to be C-076 A1a 4''-O-acetate.

## Example 2

*C-076 A2a 4'' O-Propionate*

25 Mg. of C-076 A2a is combined with 15 drops of dry pyridine and cooled in ice while 5 drops of propionic anhydride is added. The reaction mixture is stoppered, mixed well and allowed to stand in an ice bath overnight. The reaction mixture is diluted with ether and benzene and shaken with some ice water. The layers are separated and the organic layer is dried over magnesium sulfate. The solvent is evaporated under a stream of nitrogen, benzene is added and the solution is lyophilized affording 20 mg. of a white solid. Preparative layer chromatography on silica gel eluting with 5% tetrahydrofuran in chloroform affords 16.6 mg. of a white solid which is analysed by nuclear magnetic resonance and mass spectrometry as C-076 A2a 4''-O-propionate.

## Example 3

*4''-O-(Methoxycarbonyl) C-076 A2a*

25 Mg. of C-076 A2a is combined with 0.25 ml. of dry methylene chloride, 0.5 ml. of dry triethylamine and 25 mg. of 4-dimethylamin pyridine. A separate solution is prepared by adding 0.2 ml. of methylchlorocarbonate dropwise to 3 ml. of dry methylene chloride at room temperature. 0.3 Ml. of the solution is added to the first solution and the combined solutions stoppered and stirred at room temperature for 5 hours, whereupon another 0.3 ml. of the methylchlorocarbonate solution is added and the reaction mixture allowed to stir at room temperature overnight. The reaction mixture is diluted with 7.5 ml. of ether and washed 4 times with 3 ml. of water. The organic layer is dried over magnesium sulfate, concentrated *in vacuo* and dried under high vacuum affording a solid material. Preparative layer chromatography on silica gel of the solid material, eluting twice with 5% tetrahydrofuran in chloroform affords 8.2 mg. of a white solid which mass spectrometry and nuclear magnetic resonance reveals to be 4''-O-(methoxycarbonyl) C-076 A2a.

## Example 4

*4''-O-(p-Bromobenzoyl) C-076 A2a*

250 Mg. of C-076 A2a is dissolved in a mixture of 2.5 ml. of dry methylene chloride, 5 ml. of dry triethylamine and 250 mg. of 4-dimethylaminopyridine. The solution is stirred at room temperature while 3 ml. of a solution formed from 500 mg. of p-bromobenzoyl chloride and 3 ml. of dry methylene chloride is added. Another 10 ml. of methylene chloride is added and the reaction mixture is stirred for 2 hours. The reaction mixture is diluted with 200 ml. of ether and washed with a saturated sodium bicarbonate solution, 2.5 N hydrochloric acid solution, saturated sodium bicarbonate solution and water. The organic layer is dried over magnesium sulfate, concentrated *in vacuo* and dried under high vacuum affording 425 mg. of a yellow solid. Preparative layer chromatography on silica gel of the solid material, eluting twice with 5% isopropanol in benzene and once with 5% tetrahydrofuran in chloroform affords 277.4 mg. of a pale yellow solid, which mass spectrometry and nuclear magnetic resonance reveal to be 4''-O-(p-bromobenzoyl)-C-076 A2a.

Example 5

*4''-23-Di-O-(2,2,2-Trichloroethoxycarbonyl)-C-076 A2a*

A solution of 10 mg. of C-076 A2a is combined with 10 drops of dry pyridine, mixed well and 2 drops of 2,2,2-trichloroethylchlorocarbonate is added affording an immediate off-white precipitate. The reaction mixture is allowed to stand for one and a half hours. The reaction mixture is combined with ice, mixed well, allowed to stand for 20 minutes and the liquid decanted from a gummy residue. The residue is washed once with water and dissolved in 4 ml. of ether. The ether is dried over magnesium sulfate and evaporated to dryness under a stream of dry nitrogen 1.5 ml. of benzene is added and the solution is lyophilized. The lyophilized solid is dissolved in methylene chloride and purified with preparative layer chromatography on silica gel eluting with 10% tetrahydrofuran in chloroform affording 10.2 mg. of an off-white solid which mass spectrometry and nuclear magnetic resonance reveal to be 4'', 23 di-O(2,2,2-trichloroethoxycarbonyl) C-076 A2a.

Example 6

*C-076 A2a 4''-O-Benzoate*

10 Mg. of C-076 A2a is dissolved in 0.4 ml. of dry pyridine and 50 mg. of benzoic anhydride is added and the reaction vessel immersed in an oil bath at 100°C and stirred for 4 hours. The solution is allowed to cool to room temperature and combined with benzene and lyophilized affording a lyophilized powder. Preparative layer chromatography on silica gel eluting with 10% tetrahydrofuran in chloroform affords 7.8 mg. of a fluffy white solid which mass spectrometry and nuclear magnetic resonance reveal to be C-076 A2a 4'-benzoate.

Example 7

*4''-O-(Chloroacetyl) C-076 A2a*

10 Mg. of C-076 A2a is dissolved in 0.15 ml dry pyridine, cooled in an ice bath and 30 mg. of chloroacetic ahydride is added maintaining the temperature at 0°C. The reaction mixture is worked up using lyophilization and preparative layer chromatography techniques as previously described affording 6.4 mg. of a white powder which nuclear magnetic resonance and mass spectrometry reveal to be 4''-O-(chloroacetyl) C-076 A2a.

Example 8

*4''-O-(Carbomethoxypropionyl) C-076 A2a*

10 Mg. of C-076 A2a is dissolved in 0.25 ml. of a solution prepared from 108 mg. of 4-dimethylaminopyridine and 0.15 ml. of diisopropylethylamine brought to a volume of 0.5 ml. with methylene chloride. The solution is cooled in an ice bath and 0.1 ml. of methylene chloride containing 5 mg. of carbomethoxypropionyl chloride is added. The reaction mixture is stoppered and allowed to stand in an ice bath for 20 minutes. The reaction mixture is diluted with methylene chloride, washed 3 times with water, dried over magnesium sulfate and evaporated to dryness in a stream of nitrogen. Preparative layer chromatography on silica gel eluting with 15% tetrahydrofuran in chloroform affords 6.9 mg. of a white solid which mass spectrometry reveals to be 4''-O-(carbomethoxypropionyl) C-076 A2a.

Example 9

*4''-O-(N-Acetylglycyl) C-076 A2a*

30 Mg. of C-076 A2a is combined with 0.75 ml. of a solution consisting of 216 mg. of dimethylaminopyridine and 0.3 ml. of diisopropylethylamine brought to a total volume of 10 ml. with methylene chloride. This solution is cooled in an ice bath and a solution of 13.4 mg. of N-acetylglycyl chloride in 0.3 ml. of methylene chloride is added dropwise. The reaction mixture is stirred in an ice bath for 1/2 hour and at room temperature for 1 1/2 hours. The reaction mixture is worked up as previously described using preparative layer chromatography on silica gel eluting with 15% tetrahydrofuran in chloroform affording 3.2 mg. of a white solid which mass spectrometry reveals to be 4''-O-(N-acetylglycyl) C-076 A2a.

Example 10

*4''-O-(p-Nitrophenoxycarbonyl) C-076 A2a and 4'',23-di-O-(p-Nitrophenoxycarbonyl) C-076 A2a*

100 Mg. of C-076 A2a is dissolved in 2.5 ml. of a solution of 108 mg. of 4-dimethylaminopyridine and 0.15 ml. of diisopropylethylamine brought to a volume of 5 ml. with dry methylene chloride. The reaction mixture is stirred in an ice bath and another 1.0 ml. of methylene chloride is added along with 66 mg. of p-nitrophenylchloroformate. The reaction mixture is stirred at room temperature overnight and diluted with 50 ml. of ether and washed 4 times with pH 5 buffer, dried over magnesium sulfate and concentrated *in vacuo.* The residue is chromatographed on silica gel chromatography plates eluting twice with 3% tetrahydrofuran in chloroform affording two separate areas of product. There is obtained 40.6 mg. of 4'',23-di-O-(p-nitrophenoxycarbonyl) C-076 A2a and 69.8 mg. of 4''-O-(p-nitrophenoxycarbonyl) C-076 A2a.

Example 11

*C-076 A2a 4''-O-Acetate*

Following the procedure of Example 1, 5 mg. of C-076 A2a is acetylated affording 4.4 mg. of a product which is demonstrated by mass spectrometry and nuclear magnetic resonance to be C-076 A2a 4''-O-acetate.

Example 12

*C-076 A2a 4'',23-di-O-acetate*

A. 10 Mg. of C-076 A2a is acetylated in 0.5 ml. of pyridine and 0.25 ml. acetic anhydride at 100°C for 2 hours. The reaction mixture is worked up using preparative layer chromatography on silica gel as previously described affording 5.9 mg. of a fluffy white solid which mass spectrometry and nuclear magnetic resonance reveal to be C-076 A2a 4'',23-di-O-acetate.

B. The reaction part A above is repeated on 400 mg. of C-076 A2a affording 420 mg. of product which is identified analytically as C-076 A2a 4'',23-di-O-acetate.

Example 13

*C-076 B1a 4'',5-di-O-acetate*

Following the procedure of Example 1, 5.2 mg. of C-076 B1a is acetylated with 10 drops of pyridine and 6 drops of acetic anhydride affording, after preparative layer chromatography and lyophilization, 5.2 mg. of a white fluffy solid which mass spectrometry indicates is C-076 B1a 4'',5-di-O-acetate.

Example 14A

*C-076 B1a 4'',O-Acetate and C-076 B1a 4'',5-Di-O-Acetate*

20 Mg. of C-076 B1a is dissolved in 12 drops of pyridine, cooled in an ice bath and combined with 4 drops of acetic anhydride. The reaction mixture is maintained in an ice bath for 2 1/2 hours, chilled benzene is added, the reaction mixture freeze dried and the solid material chromatographed on silica gel plates eluting with 10% isopropanol in benzene. The product with the highest Rf is identified by mass spectrometry as C-076 B1a 4'',5-di-O-acetate, 4.7 mg. is obtained. The next most advanced spot is identified by mass spectrometry as C-076 B1a 4'',O-acetate; 9.3 mg. is obtained.

Example 14B

*C-076 B1a 4'',O-Acetate and C-076 B1a 4'',5-Di-O-Acetate*

The procedure of Example 14A is repeated using 500 mg. of C-076 B1a, 4.5 ml. of dry pyridine and 0.5 ml. of acetic anhydride. The reaction mixture is maintained at 0°C for 4 hours. The reaction mixture is added to a stirred ice-water mixture to obtain a white precipitate, which is filtered and the filter cake dissolved in ether. The ether layer washed twice with saturated sodium bicarbonate, once with water, dried over magnesium sulfate and concentrated *in vacuo.* The residue is placed on a column of 33 g. of silica gel and eluted with 20% ethyl acetate in methylene chloride. The fractions are collected at a rate of 4 ml. per minute and 20 ml. fractions are collected. Fractions 1—6 are discarded. Fractions 7—16 are combined and concentrated affording 122.8 mg. of a white solid. High pressure liquid chromatographic analysis of this fraction indicates the produce to be C-076 B1a 4'',5-di-O-acetate. Fractions 19—45 are also collected and treated in a similar manner affording 175.1 mg. of a white solid identified as C-076 B1a 4'',O-acetate.

Example 15

*C-076 B1a 4''-O-Acetate and C-076 B1a 4'',5-Di O-Acetate*

4.1 G. of C-076 B1a is dissolved at 37 ml. of dry pryidine and cooled in an ice bath. 4.1 Ml. of acetic anhydride is added and the reaction mixture stirred in an ice bath for 3 1/2 hours. The reaction mixture is added to a stirred mixture of 350 ml. of ice and water to obtain a white precipitate which is filtered, washed with water and dissolved in 300 ml. of ether. The ether solution is washed twice with 25 ml. portions of saturated sodium bicarbonate and once with 25 ml of water. The ether is dried over magnesium sulfate and concentrated to dryness *in vacuo* affording 4.5 g. of a white solid. The above procedure is repeated on 5.0 g. of C-076 B1a to afford 5.5 g. of a white solid, 2.6 g. of which is combined with the above 4.5 g. and the combined 7.1 g. of solid is placed on a high pressure liquid chromatography column and eluted with 25% ethyl acetate in methylene chloride at 300 ml. per minute. A forecut of 1.8 l. is taken and discarded. The next fraction of 1.6 l. is evaporated to dryness affording 1.50 g. of C-076 B1a 4'',5-di-O-acetate. Fraction 3 (300 ml.) contains 100 mg. of a mixture of C-076 B1a 4'',5-di-O-acetate, and C-076 B1a 4''-O-acetate. Fractions 4, 5 and 6 (3.0 l.) are combined and evaporated affording 3.3 g. of C-076 B1a 4''-O-acetate.

Example 16

*C-076 B1a 4''-O-Acetate and C-076 B1a 4'',5-Di-O-Acetate and C-076 B1a 5-O-Acetate*

2 G. of C-076 B1a is dissolved in 18 ml. of dry pyridine and cooled in an ice bath. 2 Ml. of acetic anhydride is added and the reaction mixture stirred in an ice bath for 3 hours and 45 minutes. The

reaction mixture is added dropwise to 300 ml. of a stirred ice and water mixture affording a white precipitate. The suspension is filtered, and the solid material washed twice with water and dissolved in 200 ml. of ether. The ether layer is washed twice with 20 ml. portions of saturated sodium bicarbonate and once with a 20 ml. portion of water. The ether is dried over magnesium sulfate, and evaporated to dryness *in vacuo* affording 2.0 g. of a white solid. The solid material is placed on a column of 130 g. of silica gel and eluted with 20% ethyl acetate in methylene chloride taking 20 ml. fractions at a rate of 7 ml. per minute. Fractions 7—44 are combined and evaporated to afford 413.9 mg. of a solid identified as C-076 B1a 4″,5-di-O-acetate. Fractions 45—48 contains less than 20 mg. of a mixture and is discarded. Fractions 49—115 are combined and contain 809.8 mg. of C-076 B1a 4″-O-acetate. Fractions 116—190 contain 246.5 mg. of a mixture which is further purified using preparative layer chromatography on silica gel plates eluting with 8% tetrahydrofuran in chloroform to afford 180.9 mg. of a solid which is again chromatographed on similar plates eluting with 10% tetrahydrofuran in chloroform affording 136.2 g. of C-076 B1a 5-O-acetate.

Example 17

*C-076 B1b 4″-O-Acetate and C-076 B1b 4″,5-di-O-acetate*

10 Mg. of C-076 B1b is dissolved in 9 drops of dry pyridine and cooled in an ice bath while 1 drop of acetic anhydride is added. The product is isolated by pouring the reaction mixture onto ice water as described in Example 17 and preparatively chromatographing the solid material on silica gel plates eluting with 8% tetrahydrofuran in chloroform affording 0.9 mg. of a material identified as C-076 B1b 4″,5-di-O-acetate and 3.2 mg. of C-076 B1b 4″-O-acetate.

Example 18

*C-076 B2a 4″,O-Acetate and C-076 B2a 4″,5-Di-O-Acetate*

200 Mg. of C-076 B2a is dissolved in 2.1 ml. of dry pyridine and cooled in an ice bath. 0.7 Ml. of acetic anhydride is added and the reaction mixture stirred for one hour. The reaction mixture is poured onto 50 ml. of ice and water with stirring. The precipitate is filtered and the solid material dried, dissolved in ether and the ether washed with water and dried over magnesium sulfate. The ether layer is evaporated to dryness and the solid material purified by preparative layer chromatography on silica gel plates eluting with 10% isopropanol in benzene affording 115.7 mg. of C-076 B2a-4″-O-acetate and 27.7 ml. of C-076 B2a 4″,5-di-O-acetate identified by mass spectrometry.

Example 19

*C-076 B2a 4″,5-Di-O-Acetate*

20 Mg. of C-076 B2a is dissolved in 12 drops of dry pyridine and cooled in an ice bath. 4 Drops of acetic anhydride is added and the reaction mixture is allowed to stand at 0°C overnight. The reaction mixture is combined with benzene and lyophilized and the solid material purified by preparative layer chromatography on silica gel plates eluting with 5% tetrahydrofuran in chloroform affording 20.8 mg. of a white solid identified by mass spectrometry as C-076 B2a 4″,5-di-O-acetate.

Example 20

*C-076 B2a 4″,5,23-Tri-O-Acetate*

20 Mg. of C-076 B2a is dissolved in 0.8 ml. of dry pyridine and 0.4 ml. of acetic anhydride is added. The reaction mixture is stirred at 100°C for 2 hours. Upon cooling, benzene is added and the mixture is lyophilized affording 22.6 mg. of a light brown solid which is purified by preparative layer chromatography on silica gel plates eluting with 5% tetrahydrofuran in chloroform affording 13.5 mg. of C-076 B2a 4″,5,23-tri-O-acetate.

Example 21

*C-076 B1a 4″,O-Propionate*

25 Mg. of C-076 B1a is dissolved in 15 drops of dry pyridine and cooled in an ice bath. 5 Drops of propionic anhydride is added and the reaction mixture stirred in an ice bath for 2 1/2 hours. The reaction mixture is added to ice chips and mixed well and ether is added. The ether layer is separated and the aqueous layer re-extracted with ether. The combined ether layers are washed with water, dried and evaporated under a stream of nitrogen affording 25 mg. of an off white solid. The solid material is purified by preparative layer chromatography on silica gel plates eluting with 10% tetrahydrofuran in chloroform affording 4.6 mg. of C-076 B1a 4″-O-propionate.

Example 22

*4″,O-(p-Chlorobenzoyl) C-076 B1a and 5-O-(p-Chlorobenzoyl) C-076 B1a*

10 Mg. of C-076 B1a is dissolved in 0.2 ml. of triethylamine and 10 mg. 4-dimethylaminopyridine is added. 0.1 Ml. of dry methylene chloride is added in orde to dissolve all of the reactants. 0.02 Ml. of p-chlorobenzoyl chloride is added. The reaction mixture is allowed to stand at room temperature for 1 1/2 hours. 6 ml. of methylene chloride and 0.6 ml. of saturated sodium bicarbonate solution is added and shaken. The aqueous layer is removed and 0.6 ml. of water is added,

shaken and separated. The ether layer is dried over mangesium sulfate and evaporated to dryness under a stream of nitrogen. Preparative layer chromatography on silica gel plates of the residue eluting with 5% isopropanol in benzene affords 5.1 mg. of a fluffy white solid identified by mass spectrometry as 4″,O-(p-chlorobenzoyl) C-076 B1a. There is also obtained 6.2 mg. of a fluffy white solid identified by mass spectrometry as 5-O-(p-chlorobenzoyl) C-076 B1a. The structures are confirmed by nuclear magnetic resonance.

Example 23

*4″-O-(3-Carbomethoxypropionyl) C-076 B1a*

10 Mg. of C-076 B1a is dissolved in 0.25 ml. of a solution of 108 mg. of 4-dimethylaminopyridine and 0.15 ml. of diisopropylethylamine in 5 ml. of dry methylene chloride. The mixture is cooled in an ice bath and 0.1 ml. of methylene chloride containing 5 mg. of carbomethoxypropionyl chloride is added. The reaction mixture is stirred at room temperature for 15 minutes and a few ice chips are added. The reaction mixture is diluted with methylene chloride, washed 34 times with water and the organic layer dried over magnesium sulfate and evaporated under a stream of nitrogen affording 10 mg. of a yellow solid. Preparative layer chromatography on silica gel plates of the residue, eluting with 5% tetrahydrofuran in chloroform affords 4.5 mg. of a solid material identified by nuclear magnetic resonance and mass spectrometry as 4″-O-(3-carbomethoxypropionyl) C-076 B1a.

Example 24

*C-076 B2a 4″-O-Propionate*

Following the procedure of Example 21 using 25 mg. of C-076 B2a there is obtained, after preparative layer chromatography on silica gel plates eluting with 10% tetrahydrofuran in chloroform, 5.1 mg. of a white solid identified by mass spectrometry as C-076 B2a 4″-O-propionate.

Example 25

*4″-O-(p-Chlorobenzoyl) C-076 B2a*

Following the procedure of Example 22 using the same scale there is obtained after preparative layer chromatography on silica gel plates eluting with 5% tetrahydrofuran in chloroform 6.4 mg. of a fluffy white solid identified by mass spectrometry as 4″-O-(p-chlorobenzoyl) C-076 B2a.

Example 26

*C-076 A2a Aglycone 13,23-Di-O-Acetate*

25 Mg. of C-076 A2a aglycone is dissolved in 0.4 ml. of dry pyidine, mixed and combined with 0.2 ml. of acetic anhydride and stirred in an oil bath at 100°C for 2 1/2 hours. The reaction mixture is cooled and combined with ice and shaken with ether. The ether layer is separated and the aqueous layer extracted twice more with ether. The ether layers are combined washed with water, dried and evaporated to dryness under a stream of nitrogen. The residue is purified by preparative layer chromatography on silica gel plates eluting with 8% tetrahydrofuran in chloroform affording 21.2 mg. of an off white solid identified by mass spectrometry as C-076 A2a aglycone 13,23-di-O-acetate.

Example 27

*13,O-(p-Chlorobenzoyl) C-076 A2a Aglycone and 23, O-(p-Chlorobenzoyl) C-076 A2a Aglycone*

21 Mg. of C-076 A2a aglycone is dissolved in 0.28 ml. of a solution prepared from 82 mg. of dimethylaminopyridine, 0.12 ml. of diisopropylethylamine and 2.5 ml. of dry methylene chloride. The mixture is stirred and cooled in an ice bath. Dropwise, 0.1 ml. of a solution of 0.065 mg. of p-chlorobenzoyl chloride in 1 ml. of dry methylene chloride is added and the reaction mixture stoppered and stirred in a ice bath for 2 hours. The reaction mixture is diluted with 12 ml. of ether and washed successively with 1.5 ml. portions of water, 2.5 normal HCl, saturated sodium bicarbonate and water. The ether layer is dried over magnesium sulfate and evaporated to dryness under a stream of nitrogen. Benzene is added and the solution lyophilized affording 40 mg. of a yellowish solid. The solid material is purified by preparative layer chromatography on silica gel plates eluting with 5% tetrahydrofuran in chloroform affording 1.2 mg. of a white solid identified by mass spectrometry as 23-O-(p-chlorobenzoyl) C-076 A2a aglycone and 12.4 mg. of a white solid also identified by mass spectrometry as 13-O-(p-chlorobenzoyl) C-076 A2a aglycone.

Example 28

*C-076 A2a Aglycone 13-O-Benzoate*

10 Mg. of C-076 A2a aglycone is dissolved in 0.3 ml. of dry pyridine and combined with 20 mg. of benzoic anhydride and heated in an oil bath at 100°C for 16 hours. The reaction mixture is cooled, benzene is added and the solution lyophilized. The residue is purified by preparative layer chromatography on silica gel plates eluting with 3% isopropanol and benzene affording 8.3 mg. of a white solid identified by mass spectrometry and nuclear magnetic resonance as C-076 A2a aglycone 13,O-benzoate.

### Example 29

*13,23-Di-O-(p-Bromobenzoyl) C-076 A2a Aglycone*

10 Mg. of C-076 A2a aglycone is combined with 0.1 ml. of dry methylene chloride, 0.2 ml. of dry triethylamine and 10 mg. of 4-dimethylaminopyridine. Dropwise, 20 ml. of p-bromobenzoyl chloride in 0.12 ml. of methylene chloride is added followed by an additional 0.4 ml. of methylene chloride to aid in dissolving the reagents. The reaction mixture is stirred at room temperature for 2 days and diluted with 10 ml. of ether and extracted once with saturated sodium bicarbonate, once with 0.5 ml. of 2.5 normal hydrochloric acid, again with sodium bicarbonate solution and then with dilute sodium chloride solution. The ether layer is dried over magnesium sulfate and evaporated to dryness under a stream of nitrogen. Preparative layer chromatography on silica gel plates eluting with 2.5% tetrahydrofuran in chloroform affords 9.5 mg. of a white solid identified by mass spectrometry and nuclear magnetic resonance as 13,23-di-O-(p-bromobenzoyl) C-076 A2a aglycone.

### Example 30

*C-076 A2a Monosaccharide 4'-O-acetate*

25 Mg. of C-076 A2a monosaccharide in 12 drops of dry pyridine is cooled in an ice bath and combined with 45 drops of acetic anhydride. The reaction mixture is stirred in an ice bath for 2 hours. Ice and water is added and the mixture extracted twice with ether and the combined ether extracts washed with water, dried over magnesium sulfate and evaporated to dryness under a stream of nitrogen. Benzene is added and the solution is lyophilized. Preparative layer chromatography on silica gel plates eluting with 10% tetrahydrofuran in chloroform affords 13.0 mg. of a white solid identified by mass spectrometry and nuclear magnetic resonance as C-076 A2a monosaccharide 4'-O-acetate.

### Example 31

*4'-O-(p-Chlorobenzoyl) C-076 A2a Monosaccharide*

25 Mg. of C-076 A2a monosaccharide is dissolved in 0.25 ml. of a solution prepared from 82 mg. of dimethylaminopyridine and 0.12 ml. of diisopropylethylamine in 2.5 ml. of methylene chloride. The reaction mixture is cooled and combined with a 0.1 ml. solution derived from 0.65 ml. of p-chlorobenzoyl chloride and 1 ml. of dry methylene chloride. The reaction mixture is stirred in an ice bath for 25 minutes. 10 Ml. of chilled ether is added and the reaction mixture washed with 2 ml. each of water, 2.5 N hydrochloric acid, saturated sodium bicarbonate solution and water. The ether layer is dried over magnesium sulfate and evaporated in a stream of nitrogen. Preparative layer chromatography on silica gel plates eluting with 5% isopropanol in benzene affords 18.5 mg. of a white powder identified by mass spectrometry as 4'-O-(p-chlorobenzoyl) C-076 A2a monosaccharide.

### Example 32

*C-076 B1a Monosaccharide 4'-O-Acetate and C-076 B1a Monosaccharide 4'-5-Di-O-Acetate*

25 Mg. of C-076 B1a monosaccharide in 12 drops of pyridine is stirred and cooled in an ice bath and combined with 4 drops of acetic anhydride. The reaction mixture is stoppered and stirred in an ice bath for 75 minutes. 50 Ml. of ice water is added and the mixture is filtered and the solid material washed with water and dissolved in 60 ml. of ether. The ether layer is washed with water, dried over magnesium sulfate and evaporated to dryness *in vacuo* affording 25 mg. of a solid material. The solid material is purified by preparative layer chromatography on silica gel plates eluting with 5% tetrahydrofuran in chloroform affording 2.2 mg. of C-076 B1a monosaccharide 4'-5-di-O-acetate and 3.8 mg. of C-076 B1a monosaccharide 4'-O-acetate identified by mass spectrometry.

### Example 33

*4'-O-(p-Chlorobenzoyl) C-076 B1a Monosaccharide*

25 Mg. of C-076 B1a monosaccharide is dissolved in 0.25 ml. of a solution derived from 82 mg. of dimethylaminopyridine, 0.12 ml. of diisopropylethylamine in 2.5 ml. of methylene chloride. The mixture is stoppered and stirred in an ice bath and combined with 0.1 ml. of a solution derived from 0.65 ml. of p-chlorobenzoyl chloride and 1 ml. of dry methylene chloride. The reaction mixture is stirred in an ice bath for 40 minutes. 10 Ml. of ether is added and the mixture washed with 2 ml. each of water, 2.5 N hydrochloric acid, saturated sodium bicarbonate solution and water. The ether layer is dried over magnesium sulfate and evaporated to dryness affording 30 mg. of a pale yellow solid. The solid material is purified by preparative layer chromatography on silica gel plates eluting with 4% tetrahydrofuran in chloroform affording 3.2 mg. of a white solid identified by mass spectrometry and nuclear magnetic resonance as 4'-O-(p-chlorobenzoyl) C-076 B1a monosaccharide.

### Example 34

*C-076 A2a 4''-Octanoate*

25 Mg. (0.028 moles) of C-076 A2a is dissolved in 0.5 ml. of methylene chloride containing 13.7 mg. of 4-dimethylamino pyridine and 14.5 mg. of diisopropyl ethylamine. This solution is cooled in an ice bath and 0.1 ml. a solution of methylene chloride containing 13.7 mg. of octanoyl chloride is added. The reaction is stirred for 40 minutes. Ice chips are added and the mixture stirred until they melted. The

mixture is then extracted twice with ether the combined organic layers washed with water dried over magnesium sulfate and evaporated to dryness under a stream of nitrogen. Preparation layer chromatography on silica gel, eluting with 3% tetrahydrofuran, 0.3% ethanol in methylene chloride affords 20.5 mg. of a solid substance which mass spectrometry confirms is C-076 A2a 4″-octanoate.

Example 35

*C-076 A2a 4″-pivalate*

Following the procedure of Example 34 using pivaloyl chloride in place of octanoyl chloride, there is obtained C-076 A2a 4″-pivalate.

Example 36

*C-076 A1a Aglycone*

100 Mg. of C-076 A1a is dissolved in 5 ml. of dioxane stirred and added at room temperature to a mixture of 0.1 ml. of concentrated sulfuric acid, 1.9 ml. of methanol and 3.0 ml. of dioxane. The reaction mixture is stirred overnight at room temperature. 473 Mg. of solid sodium bicarbonate is added and the mixture stirred for 20 minutes. 3 Ml. of water is added and stirred for an additional 10 minutes. The reaction mixture is concentrated and 40 ml. of chloroform is added and shaken. The aqueous layer is separated and extracted with 5 ml. of chloroform. The organic layers are combined and washed once with dilute sodium chloride solution, dried over magnesium sulfate and evaporated to dryness *in vacuo*. One-half of the residue is placed on 5 preparative layer chromatography silica gel plates and eluted with 2% methanol in chloroform affording 4 bands of material. The remainder of the material was run on 2 preparative layer chromatography plates eluting with 2% methanol in chloroform affording 4 bands similar to the first series. The second fastest bands are removed from each of the plates combined, extracted and evaporated to dryness *in vacuo* and rechromatographed on a preparative layer chromatography silica gel plate eluting with 3% tetrahydrofuran and chloroform affording 9.4 mg. of a fluffy white solid which is identified by mass spectrometry as C-076 A1a aglycone.

Example 37

*C-076 A2a Aglycone*

2 G. of C-076 A2a is combined with 40 ml. of a 1% (volume/volume) solution of concentrated sulfuric acid in methanol. The reaction mixture is stirred at room temperature for 17 hours and diluted with 300 ml. of chloroform. The mixture is washed once with 30 ml. of saturated sodium bicarbonate solution, once with 30 ml. saturated sodium chloride solution, dried over magnesium sulfate and evaporated to dryness *in vacuo.* 5 Ml. of methanol is added to the residue and allowed to stand at room temperature overnight. Cooling of the mixture in ice causes the slow precipitation of crystals. A supernatent is removed and the solid crystals washed twice with 1 ml. of cold methanol affording 340 mg. of a white solid. The mother liquor and washings are evaporated down to a volume of about 2 ml. and allowed to stand affording an additional crop of crystals. 630 Mg. of a white solid is obtained which is combined with the first batch of crystals and 8 ml. of methanol and evaporated to a volume of 2.5 ml. and allowed to stand for several hours. 910 mg. of an off white solid is obtained which mass spectrometry identifies as C-076 A2a aglycone.

Example 38

*C-076 A2a Monosaccharide*

500 Mg. of C-076 A2a is dissolved in 10 ml. of a solution of 0.1 ml. of concentrated sulfuric acid and 9.9 ml. of isopropanol. The reaction mixture is stirred at room temperature overnight. 125 M1. of chloroform is added and the mixture washed once with 10 ml. of saturated sodium bicarbonate and once with 10 ml. of water. The organic layer is dried over magnesium sulfate and evaporated to dryness *in vacuo* affording a pale yellow solid material which is dissolved in chloroform and placed on 5 preparative layer chromatography silica gel plates and eluted twice with 2% benzene in ethylacetate. The slower moving major fraction contains 367 mg. of a white powder after lyophilization from benzene which mass spectrometry and 300 MHz nuclear magnetic resonance indicates is C-076 A2a monosaccharide.

Example 39

*C-076 B1a Monosaccharide and C-076 B1a Aglycone*

2.5 Ml. of a solution consisting of 0.5 ml. of water 0.5 ml. concentrated sulfuric acid and 9.0 ml. of dioxane is added to 50 mg. of C-076 B1a and the reaction mixture stirred at room temperature for 17 hours. 50 Ml. of ether is added followed by 25 ml. of saturated aqueous sodium bicarbonate solution. The two layer mixture is shaken, the aqueous layer separated and the organic layer washed with water, dried and evaporated to dryness *in vacuo*. Benzene is added to the residue and the benzene layer is dried and lyophilized affording 60 mg. of yellow material. The material is placed on a preparative layer chromatography silica gel plate and eluted with chloroform/tetrahydrofuran in the volume ratio of 9:1 and 2 bands are observed with an Rf of 0.15 and 0.35. 300 MHz nuclear magnetic

resonance identifies the two spots as C-076 B1a monosaccharide and C-076 B1a aglycone respectively. 16 g. of each fraction is obtained.

Example 40

*C-076 B1a Monosaccharide*

100 Mg. of C-076 B1a is dissolved in 5.0 ml. of tetrahydrofuran and stirred at room temperature while 5.0 ml. of a cold aqueous solution of 10% sulfuric acid (volume/volume) is added dropwise with stirring. The reaction mixture is stirred at room temperature for 18 hours. 75 Ml. of methylene chloride and 25 ml. of saturated aqueous sodium bicarbonate is added and the layers shaken and separated. The organic layer is washed with aqueous sodium chloride solution and an equal volume of water. The organic layer is dried and evaporated to dryness *in vacuo* affording 70 mg. of a colorless oil. High pressure liquid chromatography identifies the residual oil as C-076 B1a monosaccharide.

Example 41

*C-076 B2a Aglycone*

2 G. of C-076 B2a is combined with 40 ml. of a 1% solution of concentrated sulfuric acid in methanol (volume/volume). The reaction mixture is stirred at room temperature for 17 hours. 300 Ml. of chloroform is added followed by 30 ml. of an aqueous saturated sodium bicarbonate solution. The layers are separated and the organic layer washed with 30 ml. of saturated sodium chloride solution, dried over magnesium sulfate and evaporated to dryness *in vacuo* 5 ml. of methanol is added to dissolve the residue and the mixture allowed to stand at room temperature and then cooled in an ice bath whereupon crystallization occurred. The supernatant is removed and the residue washed twice with 1 ml. portions of cold methanol and the solid crystals dried overnight and then *in vacuo* at 35°C affording 1.0 g. of white crystals. A second crop is obtained by evaporating the mother liquors to a volume of 2 ml. and allowing to stand overnight at room temperature. 2 Ml. of methanol is added and the mixture aged in an ice bath affording 140 mg. of a yellow solid. The two solid fractions are combined and dissolved in boiling methanol, about 30 ml. of methanol is required. The solution is filtered hot and concentrated to a volume of about 20 ml. *in vacuo* whereupon solids begin to precipitate. The solution is filtered hot and the solid materials washed with methanol affording 340 mg. of a white solid. The filtrates are boiled down to a volume of about 8 ml. and set aside to crystallize at room temperature affording 433 mg. of a white solid. Mass spectrometry shows the two fractions to be identical and to be identified as C-076 B2a aglycone.

Example 42

*C-076 B2a Monosaccharide and C-076 B2a Aglycone*

20 Mg. of C-076 B2a is combined with 4 ml. of a solution prepared by combining 0.1 ml. of concentrated sulfuric acid and 9.9 ml. of isopropanol. The reaction mixture is stirred at room temperature for 16 hours. 189 Mg. of sodium bicarbonate is added followed by a few drops of water. The volume is reduced to about 1/2 and 30 ml. of chloroform and 3 ml. of water is added and the mixture shaken. The layers are separated and the aqueous layer extracted with an additional 5 ml. of chloroform. The organic layers are combined, washed once with dilute sodium chloride solution, dried over sodium sulfate and magnesium sulfate and evaporated to dryness *in vacuo*. The residue is placed on two preparative layer silica gel chromatography plates and eluted twice with 5% tetrahydrofuran in chloroform. 4 Bands of material are observed and individually removed from the preparative chromatography plates. The slowest band affords 7.3 mg. of a white solid which is identified by mass spectrometry monosaccharide. The next slowest band affords 1.3 mg. of a white solid and it is identified by mass spectrometry as C-076 B2a aglycone.

Based on taxonomic studies, the microorganisms capable of producing C-076 compounds are of a new species of the genus Streptomyces, which has been named *Streptomyces avermitilis.* One such culture, isolated from soil, is designated MA-4680 in the culture collection of Merck & Co. Inc., Rahway, New Jersey. A C-076-producing sample of this culture has been deposited in the permanent culture collection of the Fermentation Section of the Northern Utilization Research Branch, U.S. Department of Agriculture at Peoria, Illinois, and has been assigned the accession number NRRL 8165. A sample of NRRL 8165 has also been deposited, without restriction as to availability, in the permanent culture collection of the American Type Culture Collection at 12301 Parklawn Drive, Rockville, Maryland 20852, and has been assigned the accession number ATCC 31,267.

The morphological and cultural characteristics of *Streptomyces avermitilis* are set forth below; Morphology; Sporophores form spirals as side branches on aerial mycelia. Spirals are compact but become more open as culture ages. Spores are in chains of more than 15 spores and are usually spherical to oval at 970X magnification. Sporulation is observed on oatmeal agar, glycerol-asparagine agar, salts-starch agar and egg albumin agar. Spore surface is smooth as seen by electron microscopy.

Oatmeal agar

| | |
|---|---|
| Vegetative growtl | Reverse — very dark brown |
| Aerial mycelium: | Powdery, brownish gray (4li)* mixed with white. |
| Soluble pigment: | Brown |

Czapek Dox agar (sucrose nitrate agar)

| | |
|---|---|
| Vegetative growth: | Poor, colorless |
| Aerial mycelium: | Scant, grayish |
| Soluble pigment: | Light grayish tan |

Egg albumin agar

| | |
|---|---|
| Vegetative growth: | Tan |
| Aerial mycelium: | Moderate, light grayish-yellow-brown (3ge)* mixed with white. |
| Soluble pigment: | Light yellowish tan |

Glycerol asparagine agar

| | |
|---|---|
| Vegetative growth: | Reverse — yellowish brown |
| Aerial mycelium: | Powdery, brownish gray (4li)* mixed with white. |
| Soluble pigment: | Light, yellowish brown |

Inorganic salts-starch agar

| | |
|---|---|
| Vegetative growth: | Reverse — grayish yellowish brown. |
| Aerial mycelium: | Powdery, light brownish gray (4ig)* edged with darker brownish gray (4li).* |
| Soluble pigment: | Light yellowish brown |

Yeast extract-dextrose + salts agar

| | |
|---|---|
| Vegetative growth: | Reverse — dark brown |
| Aerial mycelium: | Moderate, brownish white |
| Soluble pigment: | Brown |

Yeast extract-malt extract agar

| | |
|---|---|
| Vegetative growth: | Reverse — dark brown |
| Aerial mycelium: | Moderate, brownish white |
| Soluble pigment: | Brown |

Peptone-iron-yeast extract agar

| | |
|---|---|
| Vegetative growth: | Dark brown |
| Aerial mycelium: | None |
| Soluble pigment: | Dark brown to black |
| Melanin: | Positive |
| H S production | Positive |

Nutrient agar

| | |
|---|---|
| Vegetative growth: | Tan |
| Aerial mycelium: | Sparse, grayish |
| Soluble pigment: | Light brown |

Nutrient starch agar

| | |
|---|---|
| Vegetative growth: | Tan |
| Aerial mycelium: | Sparse, grayish white |
| Soluble pigment: | Light brown |
| Hydrolysis of starch: | Good |

Potato plug

| | |
|---|---|
| Vegetative growth: | Tan |
| Aerial mycelium: | Brown mixed with grayish white |
| Soluble pigment: | Grayish brown |

Loeffler's Blood serum

| | |
|---|---|
| Vegetative growth: | Grayish tan |
| Aerial mycelium: | None |
| Soluble pigment: | Some browning of medium |
| Liquefaction: | None |

Nutrient tyrosine agar:

| | |
|---|---|
| Vegetative growth: | Reverse — dark brown to black |
| Aerial mycelium: | Sparse, grayish |
| Soluble pigment: | Dark brown |
| Decomposition of tyrosine: | None |

Carbon utilization

Pridham-Gottlieb basal medium + 1% carbon source;
+ = growth; no growth as compared to negative control (no carbon source).

| | |
|---|---|
| Glucose | + |
| Arabinose | + |
| Cellulose | — |
| Fructose | + |
| Inositol | + |
| Lactose | + |
| Maltose | + |
| Mannitol | + |
| Mannose | + |

| | |
|---|---|
| Raffinose | + |
| Rhamnose | + |
| Sucrose | + |
| Xylose | + |
| **Nutrient gelatin agar** | |
| Vegetative growth: | Tan |
| Aerial mycelium: | Sparse, grayish white |
| Soluble pigment: | Light brown |
| Liquefaction of gelatin: | Good |
| **Gelatin stabs** | |
| Vegetative growth: | Brown ring |
| Aerial mycelium: | None |
| Soluble pigment: | Greenish brown |
| Liquifaction of gelatin: | Complete |
| **Skim milk agar** | |
| Vegetative growth: | Dark brown |
| Aerial mycelium: | None |
| Soluble pigment: | Dark brown |
| Hydrolysis of casein: | Good |
| **Litmus milk** | |
| Vegetative growth: | Dark brown growth ring |
| Aerial mycelium: | None |
| Color: | Dark brown |
| Coagulation and/or peptonization: | Complete peptonization; becoming alkaline (pH 8.1). |
| **Skim milk** | |
| Vegetative growth: | Dark brown growth ring |
| Aerial mycelium: | None |
| Soluble pigment: | Dark brown |
| Coagulation and/or peptonization: | Complete peptonization; becoming alkaline (pH 8.0). |

Temperature range: (Yeast extract-dextrose + salts agar)

28°C — Good vegetative growth and aerial mycelia

37°C — Good vegetative growth and aerial mycelia

50°C — No growth

Oxygen requirements: (Stab culture in yeast extract-dextrose + salts agar)

Aerobic

All readings taken after three weeks at 28°C unless noted otherwise. pH of all media approximately neutral (6.8 — 7.2) Color number designations (*) taken from Color Harmony Manual, 1958, 4th Edition Container Corporation of America, Chicago, Illinois.

A careful comparison of the foregoing data with published descriptions including Bergey's Manual of Determinative Bacteriology (Eighth Edition) of known microorganisms reveals significant differences that indicate that the microorganism should be classified as a new species. On this basis, it was designed *Streptomyces avermitilis.*

Other organisms can also be used to produce C-076, e.g. mutants obtained by mutating agents such as X-ray irradiation, ultraviolet irradiation or nitrogen mustards.

A culture of one such organism was isolated after irradiating *S. avermitilis* with ultraviolet light. A lyophilized tube and a frozen vial of this culture have been deposited in the permanent culture collection of the American Type Culture Collection, and they have been assigned the accession numbers 31272 and 31271 respectively. Slightly higher fermentation yields of C-076 have been obtained using this frozen stock as inoculum.

## Claims

1. A compound having the formula:

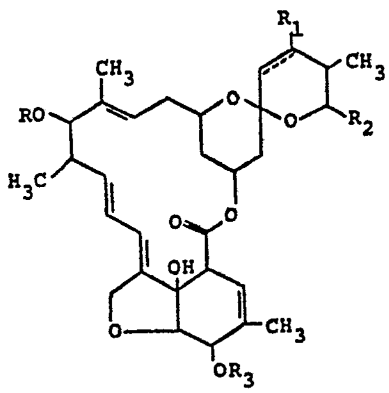

wherein the broken line indicates that the linkage is saturated or ethylenically unsaturated; $R_1$ is hydroxy or acyloxy and is present only when the broken line indicates that the said linkage is saturated; $R_2$ is *iso*-propyl or *sec*-butyl; $R_3$ is hydrogen, methyl or acyl; and R is hydrogen, acyl, $\alpha$-L-oleandrosyl, 4'-acyl-$\alpha$-L-oleandrosyl, 4'-($\alpha$-L-oleandrosyl)-$\alpha$-L-oleandrosyl or 4''-acyl-4'-($\alpha$-L-oleandrosyl)-$\alpha$-L-oleandrosyl; provided that when R is $\alpha$-L-oleandrosyl-$\alpha$-L-oleandrosyl at least one of the $R_1$ and $R_3$ groups contains an acyl group, and further that the acyl groups and the acyl portion of said acyloxy group is selected from $C_{2-12}$ alkanoyl; substituted $C_{2-12}$ alkanoyl wherein the substituents are halogen, carboxy, ($C_{1-6}$ alkoxy)carbonyl, amino; $C_{1-12}$ monoalkylamino, di-($C_{1-12}$ alkyl)amino, or $C_{2-12}$ alkanoylamino; unsaturated $C_{3-12}$ alkanoyl, ($C_{1-6}$ alkoxy)carbonyl, halogenated ($C_{1-6}$ alkoxy)carbonyl, benzoyl, or substituted benzoyl in which the substituents are halogen, nitro alkyl, amino, hydroxy or alkoxy; carbamoyl, and N-substituted and N,N-disubstituted carbamoyl wherein the substitution is $C_{1-12}$ alkyl, benzyl, $C_{1-12}$ hydroxyalkyl or the carbamoyl nitrogen may be incorporated into a morpholine heterocycle.

2. The compound of Claim 1 in which $R_2$ is *iso*-propyl.

3. The compound of Claim 1 in which $R_2$ is *sec*-butyl.

4. The compound of Claim 3 in which the acyl substituent is $C_{2-12}$ alkanoyl.

5. The compound of Claim 4 in which the $C_{2-12}$ alkanoyl group is acetyl or propionyl.

6. A method of preparing a compound as claimed in Claim 1 that comprises treating a compound having the formula:

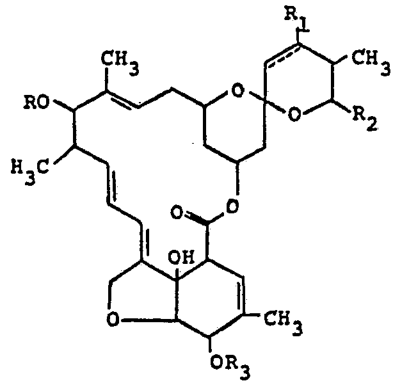

in which $R_2$ and the broken line are as defined in Claim 1, $R_1$ is hydroxy or the 22, 23 double bond is present, $R_3$ is hydrogen or methyl and R is hydrogen, α-L-oleandrosyl or 4'-(α-*L*-oleandrosyl)-α-*L*-oleandrosyl, with an acylating agent containing an acyl group as defined in Claim 1 and in the form of a halide, haloformate or anhydride.

7. A method of preparing a compound having the formula set forth in Claim 1 in which R is hydrogen or α-L-oleandrosyl, which has the formula:

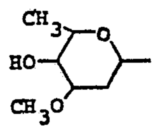

the broken line indicates that the linkage is saturated or ethylenically unsaturated: $R_1$ is hydroxy and is present only when the broken line indicates that the said linkage is saturated; $R_2$ is *iso*-propyl or *sec*-butyl; and $R_3$ is methoxy or hydroxy, that comprises treating a compound having the formula set forth in Claim 6 in which the broken line, $R_1$, $R_2$ and $R_3$ are the same as in the desired product and R is α-L-oleandrosyl-α-L-oleandrosyl, which has the formula:

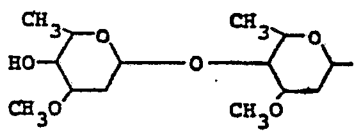

with aqueous acid in a non-nucleophilic water-miscible organic solvent in which the water is present in quantities of from 0.1 to 20% and the acid is present in quantities of from 0.01 to 0.1% by volume, to produce a compound in which R is α-*L*-oleandrosyl, or in which the acid is sulfuric acid, present in quantities of from 1 to 10% by volume, to produce the compound in which R is hydrogen.

8. A method of preparing a compound having the formula set forth in Claim 1 in which R, the broken line, $R_1$, $R_2$ and $R_3$ are as defined in Claim 7, that comprises treating a compound having the formula set forth in Claim 6 in which the broken line, $R_1$, $R_2$ and $R_3$ are the same as in the desired product and R is α-L-oleandrosyl-α-L-oleandrosyl, with 1% by volume acid in isopropanol to prepare a compound in which R is α-L-oleandrosyl, or with 1% by volume acid in methanol to prepare a compound in which R is hydrogen.

9. A compound as claimed in Claim 1 for use in the treatment of parasitic infestation.

**Revendications**

1. Un composé ayant la formule:

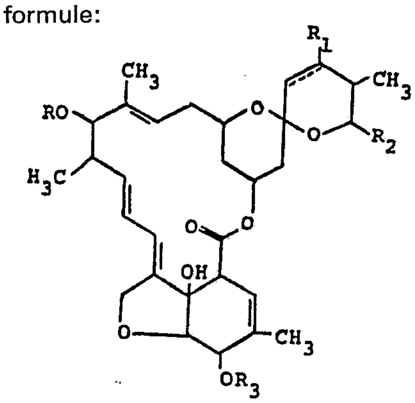

dans laquelle la ligne formée de tirets indique que la liaison est saturée ou éthyléniquement non-saturée; $R_1$ est un groupe hydroxy ou acyloxy et est présent seulement quand la ligne formée de tirets indique que ladite liaison est saturée; $R_2$ est un groupe *iso*-propyle ou *sec*-butyle; $R_3$ est de l'hydrogène ou un groupe méthyle ou acyle; et R est de l'hydrogène ou un groupe acyle, $\alpha$-L-oléandrosyle, 4'-acyl-$\alpha$-L-oléandrosyle, 4'-($\alpha$-L-oléandrosyl)-$\alpha$-L-oléandrosyle ou 4''-acyl-4'-($\alpha$-L-oléandrosyl)-$\alpha$-L-oléandrosyle; avec la condition que quand R est un groupe $\alpha$-L-oléandrosyl-$\alpha$-L-oléandrosyle, au moins un des groupes $R_1$ et $R_3$ contient un groupe acyle, et avec la condition en outre que les groupes acyle et la portion acyle du groupe acyloxy sont choisis parmi des groupes alcanoyle en $C_{2-12}$; des groupes alcanoyle en $C_{2-12}$ substitués dans lesquels les substituants sont des halogènes ou des groupes carboxy, (alcoxy en $C_{1-6}$)carbonyle, amino, mono- ou di-(alcoyle en $C_{1-12}$)-amino ou (alcanoyle en $C_{2-12}$)amino; des groupes alcanoyle en $C_{3-12}$ non saturés, (alcoxy en $C_{1-6}$)-carbonyle, (alcoxy en $C_{1-6}$)-carbonyle halogénés, benzoyle ou benzoyle substitué, où les substituants sont des halogènes ou des groupes nitro, alcoyle, amino, hydroxy ou alcoxy; des groupes carbamoyle et carbamoyle N-substitués et N,N-disubstitués dans lesquels les substituants sont des groupes alcoyle en $C_{1-12}$, benzyle, hydroxyalcoyle en $C_{1-12}$ ou l'atome d'azote du groupe carbamoyle peut être incorporé dans un hétérocycle de morpholine.

2. Un composé selon la revendication 1, dans lequel $R_2$ est un groupe *iso*-propyle.

3. Un composé selon la revendication 1, dans lequel $R_2$ est un groupe *sec*-butyle.

4. Un composé selon la revendication 3, dans lequel le substituant acyle est un groupe alcanoyle en $C_{2-12}$.

5. Un composé selon la revendication 4, dans lequel le groupe alcanoyle en $C_{2-12}$ est un groupe acétyle ou propionyle.

6. Un procédé pour préparer un composé tel que revendiqué dans la revendication 1, selon lequel on traite un composé ayant la formule:

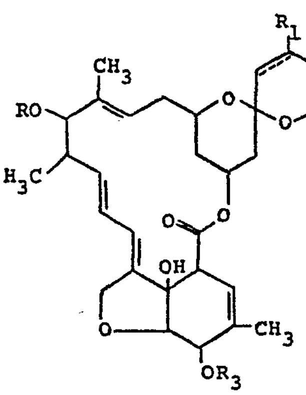

dans laquelle $R_2$ et la ligne formée de tirets sont tels que définis dans la revendication 1, $R_1$ est un groupe hydroxy ou la double liaison 22,23 est présente, $R_3$ est de l'hydrogène ou un groupe méthyle et R est de l'hydrogène ou un groupe $\alpha$-L-oléandrosyle ou 4'-($\alpha$-*L*-oléandrosyl)-$\alpha$-*L*-oléandrosyle, par un agent d'acylation contenant un groupe acyle tel que défini dans la revendication 1 et sous la forme d'un halogénure, haloformiate ou anhydride.

7. Un procédé pour préparer un composé ayant la formule spécifiée dans la revendication 1 dans laquelle R est de l'hydrogène ou un groupe $\alpha$-L-oléandrosyle, qui a la formule:

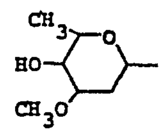

la ligne formée de tirets indique que la liaison est saturée ou éthyléniquement non saturée; $R_1$ est un groupe hydroxy et est présent seulement quand la ligne formée de tirets indique que la liaison est saturée; $R_2$ est un groupe *iso*-propyle ou *sec*-butyle; et $R_3$ est un groupe méthoxy ou hydroxy, selon lequel on traite un composé ayant la formule spécifiée dans la revendication 6 dans laquelle la ligne formée de tirets, $R_1$, $R_2$ et $R_3$ ont les mêmes significations que dans le produit désiré et R est un groupe $\alpha$-L-oléandrosyl-$\alpha$-L-oléandrosyle, qui a la formule:

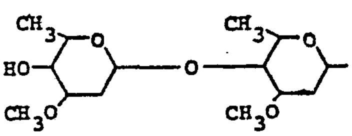

par un acide aqueux dans un solvant organique miscible avec l'eau non-nucléophile dans lequel l'eau est présente à raison de 0,1 à 20% et l'acide est présent à raison de 0,01 à 0,1% en volume, de façon à produire un composé dans lequel R est un groupe $\alpha$-L-oléandrosyle, ou dans lequel l'acide est de l'acide

sulfurique, présent à raison de 1 à 10% en volume, de manière à produire le composé dans lequel R est de l'hydrogène.

8. Un procédé pour préparer un composé ayant la formule spécifiée dans la revendication 1 dans laquelle R, la ligne formée de tirets, $R_1$, $R_2$ et $R_3$ sont tels que définis dans la revendication 7, selon lequel on traite un composé ayant la formule spécifiée dans la revendication 6 dans laquelle la ligne formée de tirets, $R_1$, $R_2$ et $R_3$ ont les mêmes significations que dans le produit désiré et R est un groupe $\alpha$-L-oléandrosyl-$\alpha$-L-oléandrosyle, par 1% en volume d'acide dans de l'isopropanol de manière à préparer un composé dans lequel R est un groupe $\alpha$-L-oléandrosyle, ou par 1% en volume d'acide dans du méthanol de manière à préparer un composé dans lequel R est de l'hydrogène.

9. Un composé tel que défini dans la revendication 1 pour application dans la lutte contre les parasites.

**Patentansprüche**

1. Verbindung mit der Formel

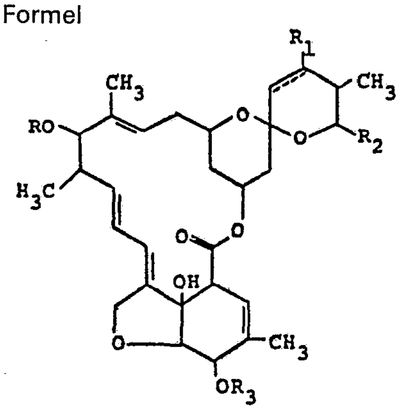

worin die unterbrochene Linie anzeigt, daß die Bindung gesättigt oder äthylenisch ungesättigt ist; $R_1$ Hydroxy oder Acyloxy ist und nur vorhanden ist, wenn die unterbrochene Linie anzeigt, daß die Bindung gesättigt ist; $R_2$ Isopropyl oder sec-Butyl ist; $R_3$ Wasserstoff, Methyl oder Acyl ist; und R Wasserstoff, Acyl, $\alpha$-L-Oleandrosyl, 4'-Acyl-$\alpha$-L-oleandrosyl, 4'-($\alpha$-L-Oleandrosyl)-$\alpha$-L-oleandrosyl oder 4''-Acyl-4'-($\alpha$-L-oleandrosyl)-$\alpha$-L-oleandrosyl ist; vorausgesetzt, daß, wenn R $\alpha$-L-Oleandrosyl-$\alpha$-L-oleandrosyl ist, mindestens eine der Gruppen $R_1$ und $R_3$ eine Acylgruppe enthält und darüber hinaus, daß die Acylgruppen und der Acylteil der Acyloxygruppe ausgewählt sind aus $C_{2-12}$-Alkanoyl; substituiertem $C_{2-12}$-Alkanoyl, worin die Substituenten Halogen, Carboxy, ($C_{1-6}$Alkoxy)-carbonyl, Amino, $C_{1-12}$-Monoalkylamino, Di-($C_{1-12}$-alkyl)-amino oder $C_{2-12}$-Alkanoylamino sind; ungesättigtem $C_{3-12}$-Alkanoyl, ($C_{1-6}$-Alkoxy)-carbonyl, halogeniertem ($C_{1-6}$-Alkoxy)-carbonyl, Benzoyl oder substituiertem Benzoyl, worin die Substituenten Halogen, Nitro, Alkyl, Amino, Hydroxy oder Alkoxy sind; Carbamoyl und N-substituiertem und N,N-disubstituiertem Carbamoyl, worin die Substitution $C_{1-12}$-Alkyl, Benzyl, $C_{1-12}$-Hydroxyalkyl ist oder der Carbamoylstickstoff in einen Morpholin-Heterozyklus einbezogen werden kann.

2. Verbindung nach Anspruch 1, worin $R_2$ Isopropyl ist.

3. Verbindung nach Anspruch 1, worin $R_2$ sec-Butyl ist.

4. Verbindung nach Anspruch 3, worin der Acylsubstituent $C_{2-12}$Alkanoyl ist.

5. Verbindung nach Anspruch 4, worin die $C_{2-12}$-Alkanoylgruppe Acetyl oder Propionyl ist.

6. Verfahren zur Herstellung einer Verbindung nach Anspruch 1, bei dem eine Verbindung mit der Formel

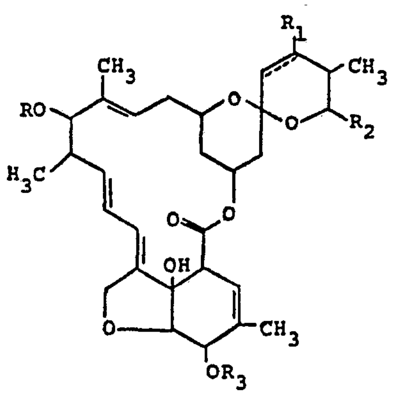

worin $R_2$ und die unterbrochene Linie wie in Anspruch 1 definiert sind, $R_1$ Hydroxy ist oder die 22, 23-Doppelbindung vorhanden ist, $R_3$ Wasserstoff oder Methyl ist und R Wasserstoff, $\alpha$-L-Oleandrosyl oder

4'-($\alpha$-L-Oleandrosyl)-$\alpha$-L-oleandrosyl ist, mit einem Acylierungsmittel, das eine Acylgruppe, wie in Anspruch 1 definiert, enthält und in der Form eines Halogenids, Halogenformiats oder Anhydrids, behandelt.

7. Verfahren zur Herstellung einer Verbindung mit der in Anspruch 1 angegebenen Formel, worin R Wasserstoff oder $\alpha$-L-Oleandrosyl ist, mit der Formel:

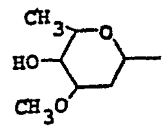

die unterbrochene Linie anzeigt, daß die Bindung gesättigt oder äthylenisch ungesättigt ist; $R_1$ Hydroxy ist und nur vorliegt, wenn die unterbrochene Linie anzeigt, daß die Bindung gesättigt ist; $R_2$ Isopropyl oder sec-Butyl ist; und $R_3$ Methoxy oder Hydroxy ist, bei dem eine Verbindung mit der in Anspruch 6 angegebenen Formel in der die unterbrochene Linie, $R_1$, $R_2$ und $R_3$ die gleichen sind wie in dem gewünschten Produkt und R $\alpha$-L-Oleandrosyl-$\alpha$-L-oleandrosyl ist, mit der Formel

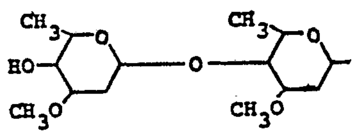

mit wäßriger Säure in einem nicht-nukleophilen mit Wasser mischbaren organischen Lösungsmittel, in dem das Wasser in Mengen von 0,1 bis 20% vorhanden ist und die Säure in Mengen von 0,01 bis 0,1%, bezogen auf das Volumen, vorhanden ist, behandelt wird, unter Bildung einer Verbindung, worin R $\alpha$-L-Oleandrosyl ist, oder worin die Säure Schwefelsäure ist, vorhanden in Mengen von 1 bis 10%, bezogen auf das Volumen, zur Herstellung der Verbindung, worin R Wasserstoff ist.

8. Verfahren zur Herstellung einer Verbindung mit der in Anspruch 1 angegebenen Formel, worin R, die unterbrochene Linie, $R_1$, $R_2$ und $R_3$ wie in Anspruch 7 definiert sind, bei dem eine Verbindung mit der in Anspruch 6 angegebenen Formel, worin die unterbrochene Linie, $R_1$, $R_2$ und $R_3$ die gleichen sind wie im gewünschten Produkt, und R $\alpha$-L-Oleandrosyl-$\alpha$-L-oleandrosyl ist, mit 1%, bezogen auf das Volumen, Säure, in Isopropanol behandelt wird, unter Bildung einer Verbindung, worin R $\alpha$-L-Oleandrosyl ist, oder mit 1%, bezogen auf das Volumen, an Säure in Methanol, unter Herstellung einer Verbindung, worin R Wasserstoff ist.

9. Verbindung nach Anspruch 1 zur Verwendung bei der Behandlung von Parasitenbefall.